# EUROPEAN PATENT SPECIFICATION

(11) **EP 2 117 639 B1**
(45) Date of publication and mention of the grant of the patent: **22.05.2013**
(21) Application number: 07709366.4
(22) Date of filing: 21.02.2007
(51) Int. Cl.: A61N 1/08, A61N 1/36, A61B 5/026, A61B 8/06

(54) **DETECT EATING TO INITIATE GASTRIC PACING**
NACHWEIS VON NAHRUNGSAUFNAHME ZUR EINLEITUNG VON MAGEN-PACING
DÉTECTION DE LA PRISE DE NOURRITURE POUR INITIER UNE STIMULATION GASTRIQUE

(43) Date of publication of application: 18.11.2009
(73) Proprietor: St. Jude Medical AB, 175 84 Järfälla (SE)
(72) Inventor: BJÖRLING, Anders, S-169 37 Solna (SE); NOREN, Kjell, S-171 58 Solna (SE); ÖHLANDER, Malin, S-118 48 Stockholm (SE)
(74) Representative: Sjölander, Henrik
(86) International application number: PCT/SE2007/000154
(87) International publication number: WO 2008/103077

(56) References cited:
- WO-A2-2004/112883
- US-A- 5 188 104
- US-A1- 2004 162 469
- US-A1- 2005 149 142
- US-A1- 2005 222 638
- US-A1- 2006 129 201
- US-A1- 2006 129 204
- GARCIA-GRANERO E ET AL: "Use of photoplethysmography to determine gastrointestinal perfusion pressure: an experimental canine model" DIGESTIVE SURGERY, S. KARGER AG, CH, vol. 20, no. 3, 1 January 2003 (2003-01-01), pages 222-228, XP009133099 ISSN: 0253-4886

## Description

### Field of the invention

The present invention relates to methods and devices for monitoring at least one physiological parameter that relates to the activity of the digestive system of a patient.

### Background of the invention

A modem major health concern is the increasing proportion of the human population who are so overweight that they suffer from obesity. Obesity is a leading cause of premature deaths and is associated with other health problems such as high blood pressure, heart disease, strokes, breathing disorders, cancer and musculoskeletal problems.

The treatment of obesity is complex as there are many factors which contribute to a person becoming obese, the major ones are considered to be lack of exercise and overeating. In order to reduce the amount that a patient eats it is known to use gastric stimulation. The theory behind this is that stimulation of the gastroenteric system (the stomach's own nervous system) or the vagal nerve between the stomach and the brain by electrical impulses will lead more quickly to the patient having a feeling of satiety and consequently the patient will stop eating earlier than he or she previously would, thereby reducing the amount of overeating of the patient. Implantable devices and methods for gastric stimulation are known from US patent application US2005/0149142. In order to avoid excessive battery draining and to increase the longevity of the implanted device it is known to stimulate the gastroenteric system only when the patient has commenced eating. US2005/0149142 teaches a number of ways in which eating by a patient can be detected by a monitoring a physiological parameter that reflects activity of the patients stomach. Such parameters including gastric electrical activity, trans-abdominal impedance, blood glucose levels, insulin levels, stomach acid levels, motion of the stomach, distension of the stomach or other mechanical indicators of stomach activity.

WO 2004/112883 describes an apparatus for detecting eating by a subject comprising a food intake detection device. The food intake detection device is adapted to be coupled to a hepatic portal vein of a subject and may comprise a blood flow sensor, which is adapted to detect a rate of blood flow in the portal vein. Increased portal blood flow is said to be indicative of elevated postprandial portal blood glucose, carbohydrate, fat, or protein levels, and thus indicates the quantity of such products recently absorbed by small intestine during eating. The blood flow sensor may comprise an implantable blood flow meter, e.g. a pulsed Doppler ultrasonic flow meter, or a meter that utilizes the thermodilution principle. Alterntaively, the blood flow sensor may comprise one or more electrodes adapted to measure changes in impedance between the electrodes, which changes are indicative of stretching of portal vein caused by increased portal blood flow.

### Summary of the invention

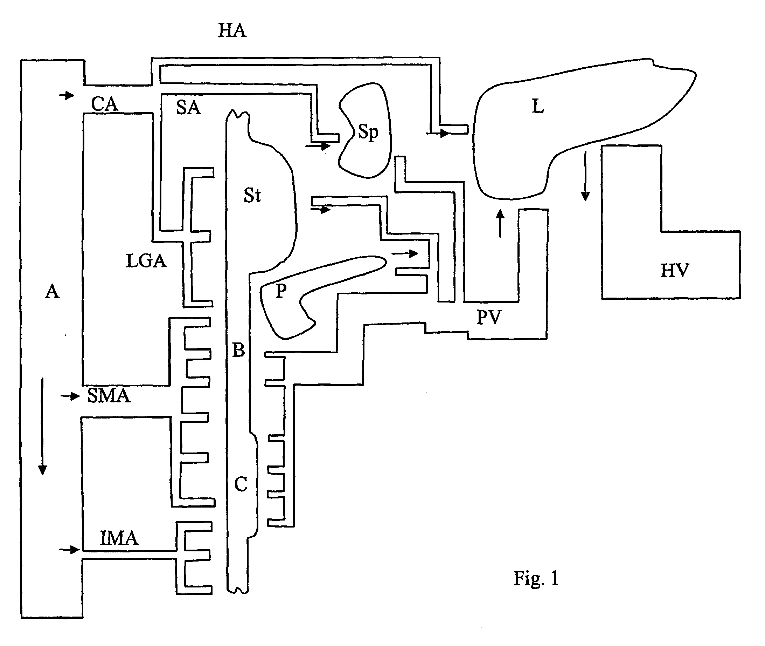

It is known that the energy consumption of the digestive system (which normally is defined as including the stomach, spleen, pancreas, liver, small bowel and colon) increases due to the increase of gastric activity which follows the ingestion of food. This increase in energy consumption is reflected, amongst others, by an increase in blood to, and perfusion of, the organs in the digestive system. As shown schematically in figure 1, the gastrointestinal tract is perfused by three branches from the aorta (A): the coeliac artery (CA) (which supplies blood to the stomach (St) via the left gastric artery (LGA), the liver (L) via the hepatic artery (HA) and the spleen (SP) via the splenic artery (SA)), the superior mesenteric artery (SMA) and the inferior mesenteric artery (IMA). These supply the stomach (St) and viscera (the splanchnic system) with a large blood flow in comparison to other organs - during fasting under basal conditions approximately 20%-30% of the cardiac output goes through the splanchnic vasculature. This blood is fed to the liver (L) from the stomach (St), spleen (Sp), pancreas (P), small bowel (B) and colon (C) via the portal vein (PV) and accounts for approximately 80% of the blood flow to the liver. The remaining 20% of the blood flow to the liver is directly from the hepatic artery (HA). Blood from the liver returns to the heart via the hepatic veins (HV) to the inferior vena cava.

Typically the portal venous blood flow is 1200 ml/min in the fasting state but it may increase to 2000 ml/min following a meal. The SMA blood flow can double from 500 to 1000 ml/min within 15 minutes of food ingestion, the increase being dependent on caloric load, food volume and type. Similarly the CA blood flow can increase from 800 ml/min to 1100 ml/min following feeding.

The present invention is as per claim 1. Although, as stated above, normally the digestive system is defined to include the stomach, spleen, pancreas, liver, small bowel and colon, for the invention described in the present patent application, changes in the blood flow or perfusion of the small bowel and colon occur too long after the ingestion of food to be useful in detecting that eating has begun. Consequently, in the following the expression "digestive system" will be taken to mean the stomach, spleen, pancreas and liver unless stated otherwise, as changes in the blood flow and perfusion of these organs occur quickly after the onset of eating.

While multiple embodiments are disclosed, still other embodiments of the present invention will become apparent to those skilled in the art from the following detailed description, which shows and describes illustrative embodiments of the invention. As will be realized, the invention is capable of modifications in various aspects, all without departing from the scope of the present invention. Accordingly, the drawings and detailed description are to be regarded as illustrative in nature and not restrictive.

### Brief description of the figures

Figure 1 is a diagram illustrating schematically the human digestive system and the important blood vessels supplying it with blood;
Figures 2a) and 2b) shows schematically a first embodiment of a device for measuring perfusion to the stomach of a patient using a photoplethysmograph in accordance with the present invention;
Figure 3 shows schematically an embodiment of an ultrasound arrangement for measuring the flow rate in a blood vessel;
Figures 4a) and 4b) show schematically an embodiment of a device for measuring the diameter of a blood vessel;
Figure 5 shows schematically a further embodiment of a device for measuring the diameter of a blood vessel;
Figure 6 shows schematically an embodiment of an electromagnetic induction device for measuring the flow rate in a blood vessel;
Figure 7 shows schematically an embodiment of a calorimetric device for measuring flow velocity in a blood vessel;
Figure 8 shows schematically an embodiment of a thermistor device for measuring flow velocity in a blood vessel;
Figure 9 shows an example of measured blood flow amplitude (F) to the stomach of a patent during a 24 hour period (T); and,
Figure 10 is a 24-bin histogram of the blood flow amplitude data shown in figure 9.

### Description of the invention

Changes in the blood flow of the digestive system of a patient can be used to determine if the patient has started to eat. These changes can be measured by taking measurements of a parameter related to blood flow directly at one or more organs or by measuring the blood flow through one or more arteries or veins connected to an organ or system of organs.

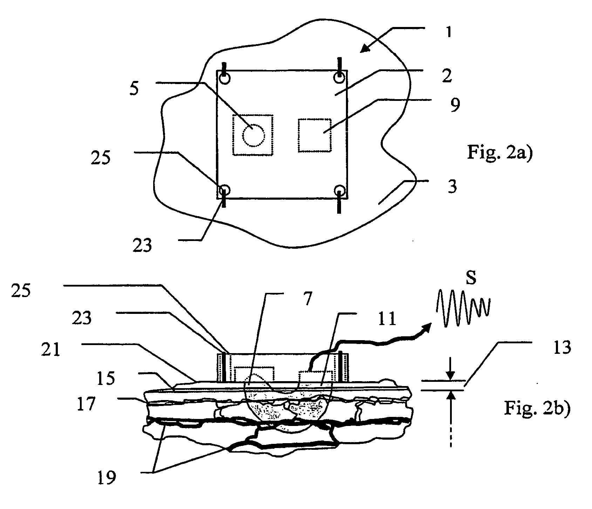

In a first embodiment of a sensing device 1 in accordance with the present invention, shown schematically in figures 2a) and 2b), a reflective photoplethysmograph (PPG) 2 is used to analyse a parameter related to blood flow of an organ of a patient, in this embodiment the perfusion of the stomach 3. Only elements of the PPG necessary for the understanding of the present invention are shown, other features such as power supply, switch(es), contacts, leads, etc being omitted for the sake of clarity of the figures. The PPG 2 comprises at least one light source, such as light emitting diode (LED) 5, which emits electromagnetic radiation 7 towards the organ being analysed. It further comprises at least one sensor, such as phototransistor 9, sensitive to the electromagnetic radiation reflected from the organ 3, which is arranged to receive the electromagnetic radiation 11 reflected from the tissues of the organ such as the epidermis 13, pigmentation 15, capillaries 17 and other blood vessels 19 of the dermis. Sensing device 1 produces an output signal S dependant on the intensity of the reflected electromagnetic radiation. The intensity of the reflected electromagnetic radiation varies according to the perfusion of the portion of the tissue of the organ 3 that the electromagnetic radiation passes through when travelling from the source 5 to the sensor 9. The output signal S can be transmitted to analytical device such as a gastric stimulator (not shown), which analyses the signal in order to detect changes in perfusion and determines from these changes when feeding has commenced. Preferably the PPG 2 is held in place on a particular part of the stomach in order to prevent artefacts caused by relative movement between the PPG and the stomach. A PPG can be sutured to the wall 21 of the stomach by sutures 23 which pass through suture holes 25 provided on the PPG 2. As an alternative, a PPG could be mounted on the stomach by means of a belt or ring that is placed around the stomach. The belt could be the same as, or similar to, a band, known in the art, used for gastric banding. In gastric banding, a belt is placed around the stomach during surgery to decrease the usable volume of the stomach. The PPG could be placed between the stomach and the belt. Similarly if a beltlike stimulation ring is arranged around the stomach for electro stimulation of the stomach, the PPG could be integrated into the stimulation ring or placed between the stimulation ring and the stomach.

In a second embodiment of a sensing device (not part of the present invention), the intramuscular resistance or impedance of the stomach of a patient is measured by means of an impedance sensing device comprising two or more electrodes spaced close to each other across a portion of the stomach wall. Impedance measuring signals of predetermined currents/voltages are transmitted from one electrode and the tissue that the signal passes though before being received at the other electrode(s) modulates the signal so that the current/voltages received at the receiving electrode(s) will have different amplitudes and phase-angles. By choosing a frequency which is sensitive to changes in the perfusion of the tissue, changes in the phase-angle and amplitude can be used to identify a change in perfusion.

In a third embodiment of a device (not part of the present invention), the resistance or impedance across a blood vessel related to the digestive system of a patient can be monitored in order to determine the flow inside it. Blood vessels in which the flow increases shortly after feeding has commenced (e.g. the left gastric artery, the hepatic artery, the portal vein, the celiac artery and the lienal artery) are preferred, as monitoring of these blood vessels allows stimulation to begin shortly after feeding has begun. The resistance of blood is lower than the resistance of other body tissues. Owing to this, increases in the flow of blood to the digestive system, and the accompanying increase in the diameter of the blood vessels through which the blood flows, lead to a change in the impedance or resistance measured. If impedance is measured instead of resistance then it should preferably be measured at a low frequency since impedance measurements at a low frequency are dominated by the resistive component of the measurement. A sensing device could comprise sensing electrodes positioned diametrically-opposed each other on a blood vessel and changes in resistance or impedance between the electrodes analysed. When the diameter of the blood vessel increases, which occurs when the blood flow through it increases, then the resistance across it changes due to changes in the average resistivity of the media between the electrodes. Furthermore, as the flow velocity increases, the orientation of the blood cells in the blood vessel changes. At low velocities the blood cells are randomly orientated but as the flow velocity increases the cells become orientated with the greatest surface area facing the stream. At measurement frequencies over 4 kHz the impedance measured inside the vessel is affected by this change in orientation and decreases as the flow velocity increase. If measurements are taken on a blood vessel at two different frequencies with different electrodes it is possible to obtain metrics related to both the blood velocity and the surface area of the blood flow. These can be combined to obtain the blood volume per unit time.

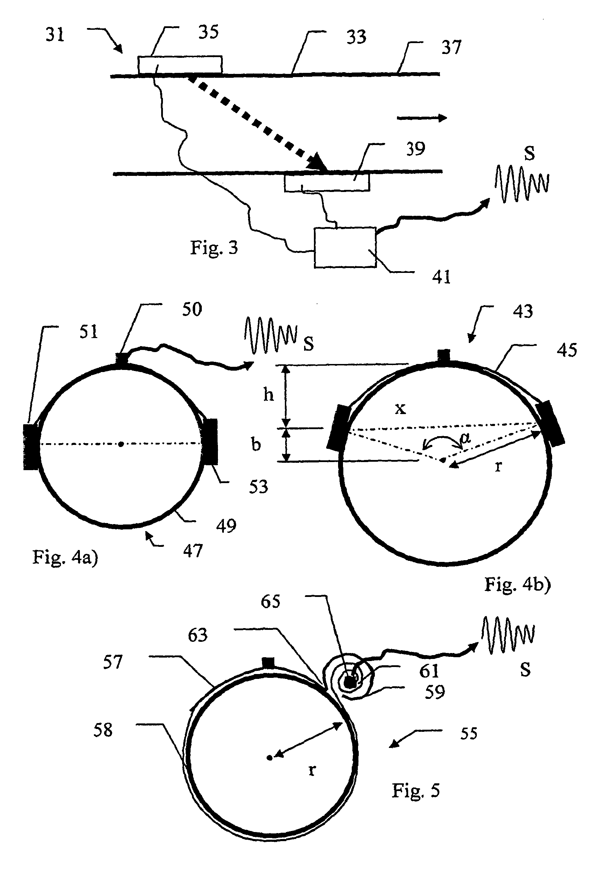

Figure 3 shows schematically an embodiment of an ultrasound sensing device 31 for measuring the flow rate in a blood vessel 33 (not part of the present invention). This sensing device comprises an ultrasound transmitter 35 attached to the wall 37 of blood vessel 33 (for example by a cuff - not shown) and an ultrasound receiver 39 attached to the wall of the blood vessel opposite transmitter 35 and either upstream or down stream of it. Ultrasound transmitter 35 is controlled by a control device 41 to emit a pulse or pulses of ultrasound at predetermined intervals and the arrival time of these pulses at receiver 39 is registered by control device 41. Owing to the Doppler Effect the time between an ultrasound pulse being transmitted from the transmitter 35 and its receipt at receiver 39 varies as the speed of the flow in blood vessel varies. Control device 41 can produce an output signal S which reflects the variations in the time between pulses being transmitted and received. Alternatively data relating to the transmitting of pulses from transmitter 35 and data relating to their arrival time at receiver 39 can be transmitted directly to a stimulating device which analyses this data to determine if feeding has commenced.

Figures 4a) and 4b) show schematically an embodiment of a sensing device 43 for measuring the diameter of a blood vessel (not part of the present invention). This device comprises a flexible cuff 45 which is placed around a blood vessel 47. Cuff 45 is flexible enough to follow movement of the wall 48 of the blood vessel 47 without restricting flow through the blood vessel, while at the same time staying in contact with the blood vessel. Cuff 45 is provided with a stretch sensor, for example a strain gauge, 50 which in positioned in the centre of the cuff. This stretch sensor 50 produces an output signal S which varies as the ends 51, 53 move closer and further apart due to changes in the diameter of the blood vessel. As increases in the diameter of a blood vessel can be assumed to reflect increases in the flow inside the vessel, data relating to the diameter of the blood vessel can be analysed to determine if eating has commenced.

Figure 5 shows schematically a further embodiment of a sensing device 55 for measuring the diameter of a blood vessel (not part of the present invention). Sensing device 55 comprises a cuff 57 which surrounds a blood vessel 58. Cuff 57 has a spring-loaded coiled end 61 which can coil and uncoil inside a housing 59 attached to the other end 63 of cuff 57. End 61 coils and uncoils to follow the diameter of the blood vessel. A sensor 65 inside housing 59 detects how much coiled end 61 moves and produces an output signal S which relates to the circumference of the blood vessel.

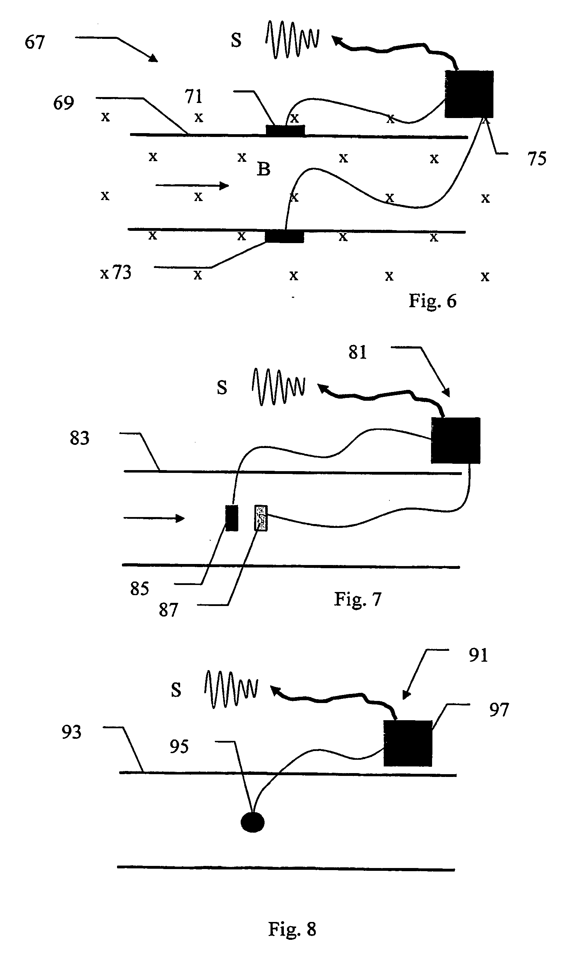

Figure 6 shows schematically an embodiment of an electromagnetic induction sensing device 67 for measuring the flow rate in a blood vessel 69 of a patient (not part of the present invention). A pair of electrodes 71, 73 are placed on opposite sides of the blood vessel and connected to a voltage measuring device 75. The blood vessel is subjected to a magnetic field B (represented by "x" in figure 6) of the order of 1 mT which acts perpendicular to the direction of blood flow. As blood flows between the electrodes 71, 73 it causes a voltage to be generated which is proportional to the average blood velocity in the blood vessel between the electrodes perpendicular to the magnetic field, the magnetic field and the distance between the electrodes. The voltage measuring device can produce an output signal S which can be used to determine the velocity of the blood in the blood vessel. The blood vessel diameter can be estimated using any suitable method and the total blood flow volume calculated.

Figure 7 shows schematically an embodiment of a calorimetric sensing device 81 for measuring flow velocity in a blood vessel 83 of a subject (not part of the present invention). A first temperature sensor 85 is mounted closely upstream of a second temperature sensor 87 in said blood vessel 83. First temperature sensor 85 is heated to a temperature which is slightly above blood temperature, e.g. 2° C above blood temperature. These sensors 85, 87 can be mounted on a lead, not shown, which contains conductors for supplying electrical energy to said second temperature sensor 87 and transmitting signals from said sensors 85, 87 to a control device (not shown). The temperature registered by downstream second temperature sensor 87 is influenced by the heat transferred to it from first temperature sensor 85. The amount of heat transferred to it from first temperature sensor 85 is influenced by the velocity of the blood flowing past it - as the blood flow increases proportionally more energy is transferred from the first sensor 85 to the second sensor 87 and an output signal S from the second sensor 87 registers an increase in temperature. The value of the output signal S can be used to determine the velocity of the blood in the blood vessel 83.

Figure 8 shows schematically an embodiment of a thermistor sensing device 91 for measuring flow velocity in a blood vessel 93 of a subject (not part of the present invention). A thermistor 95 is mounted on a lead, not shown, which contains conductors for supplying electrical energy to said thermistor 95 and transmitting signals from said thermistor 95 to a control device 97. Control device 97 supplies electrical energy to thermistor 95 so that it is maintained at a constant temperature which is slightly above blood temperature, e.g. 2° C above blood temperature. The amount of electrical energy needed to maintain this temperature depends on the amount of energy lost to the blood flowing past the thermistor and is dependent on the velocity of the blood flowing past the thermistor. Control device 97 can monitor the current supplied to thermistor 95 and produce an output signal S related to this current and which can be used to determine the velocity of the blood in the blood vessel 93.

Other sensors which can be used to determine flow rates include vortex counters and elbow flow meters. Vortex counters are based on the principle that an obstruction in a fluid flow can create vortices downstream of the obstruction. Every obstruction has a critical fluid flow rate at which vortex shedding occurs. Vortex shedding causes alternating low pressure zones downstream of the obstruction which exert a downstream pull force on the obstruction. Analysis by a control device of the frequency of the vortices and the force that the low pressure exerts on the obstacle can be used to determine the fluid flow rate. Elbow flow meters are based on the principle that a differential pressure exists when a flowing fluid changes direction due to a pipe turn or elbow - a high pressure node forms at the outside of the bend and a low pressure node forms at the inside of the bend. Pressure transducers can be attached at the inside and outside of a bend in a blood vessel and will detect different pressures due to the pressure differential at the bend. The data from the transducers can be processed by a control device to determine the fluid flow rate.

In a first method to operate a gastric stimulator a patient is provided with a sensing device which can measure the perfusion, or changes in the perfusion, of an organ in the digestive system of the patient wherein increased organ activity may indicate that the patient has begun eating, or a sensing device which can measure the blood flow, or changes in the blood flow, in a blood vessel connected to the digestive system which blood vessel exhibits an increase in flow when the patient has begun eating. The sensing device produces an output signal which reflects variations in the perfusion of, or blood flow in, the organ or blood flow in the blood vessel. This output signal may be used to control a gastric simulator. This can be achieved by providing the sensing device with, or connecting the sensing device to, control means which can analyse the output signal and cause a control signal to be sent to the gastric stimulator to start its stimulating function when a first predetermined value of blood flow or perfusion indicating that feeding has started has been reached, and to stop its stimulating function when a second predetermined value is reached which indicates that feeding has finished or some other activity has been started or a predetermined period of time has elapsed, and which indicates that further stimulation is unnecessary. Alternatively the raw output signal can be transmitted from the sensing device to the gastric stimulator which itself analyses the output signal and determines from this analysis when gastric stimulation should be started and stopped or modified.

In a second method to operate a gastric stimulator a patient is provided with two or more sensing devices, each of which can measure the perfusion of, or blood flow in, or changes in the perfusion of, or blood flow in, an organ in the digestive system of the patient wherein increased organ activity may indicate that the patient has begun eating and/or which can measure the blood flow or changes in the blood flow in a blood vessel connected to the digestive system which blood vessel exhibits an increase in flow when the patient has begun eating. The sensing devices each produce an output signal which reflects variations in the perfusion of the organ or blood flow in the blood vessel. One or more of these output signals may be used to control a gastric simulator. This can be achieved by providing the sensing devices with control means which can analyse the output signal and cause a control signal to be sent to the gastric stimulator to start its stimulating function when a first predetermined value of blood flow and/or perfusion indicating that feeding has started has been reached, and to stop its stimulating function when a second predetermined value is reached and which indicates feeding has finished or some other activity has been started or a predetermined period of time has elapsed, and that further stimulation is unnecessary. Alternatively the raw output signals can be transmitted from the sensing devices to the gastric stimulator which itself analyses the output signals and determines from this analysis when gastric stimulation should be started and stopped or modified.

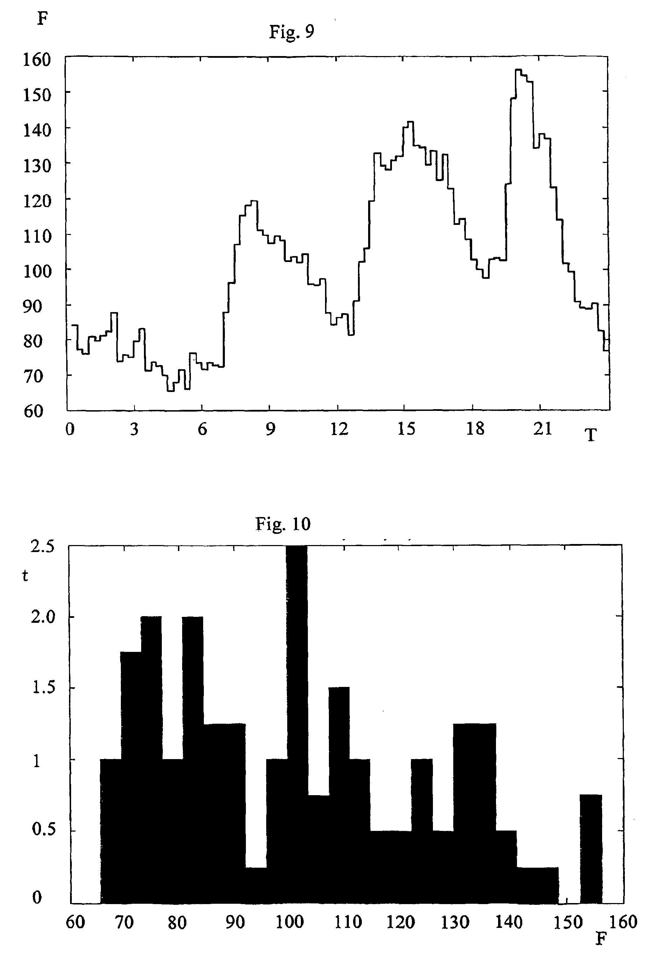

When a system for the gastric stimulation of a patient is in use, it can be of interest to keep track of how often and how much a patient is eating. As described above, one way of detecting eating is by analysing the blood flow to the stomach of the patent. Figure 9 shows an example of measured blood flow amplitude (F) to the stomach of a patent during a 24 hour period (T). The blood flow was sampled once per minute and averaged over 15 minutes. The flow amplitude is in arbitrary units, for instance as a percentage of a normal level. Three feeding occasions can be identified from the curve in figure 9: at 7 (7 am) in the morning, at 13 (1 pm) and 19:45 (7:45 pm).

One way of keeping track of how many times a day the patient is eating would be to either count the number of eating occasions per day or, if storage of that information would be too memory-consuming, to create a histogram of the number of times the patient eats per day and to refresh the histogram at regular intervals, e.g. daily.

As the amplitude and duration of increased blood flow to the stomach appears to be related to the number of calories ingested in a meal, it can be of interest to analyse the duration of increased blood flow to the stomach. Figure 10 is a 24-bin histogram of the blood flow amplitude data shown in figure 9 where amplitude of the column in each bin shows the duration in hours (t) for each blood flow amplitude (F). This histogram provides information on how much food the patient consumed during a 24-hour period. The amount of time that the blood flow to the patient's stomach at a high blood flow amplitude can be used to determine how active the stomach is and comparison of these daily histograms over a period of time can show if the patient's stomach is working less - i.e. the patient is consuming less food - or more. If, despite the use of a gastric stimulating system, analysis of the histograms show that patient is eating the same amount or more, then the stimulating regime provided by the gastric stimulating system can be modified in order to reduce the patient's intake of food.

## Claims

1. Device for detecting the intake of food in a subject **characterised in that** it comprises:
- a photoplethysmograph (2) able to be arranged on an organ of the digestive system of said subject, said organ being one of the stomach, the spleen, the pancreas and the liver, in order to measure perfusion of said organ of the digestive system and to produce an output signal dependent on said perfusion, and
- an analytical device arranged to analyze said output signal so as to detect intake of food.

2. Device in accordance with claim 1 **characterised in that** said photoplethysmograph is attachable to the stomach (3) of said subject.

3. Device in accordance with claim 1 or 2 **characterized in that** said photoplethysmograph is mountable on a belt or ring that is able to be placed around the stomach of said subject or said photoplethysmograph is provided with suture holes (25).

4. Device in accordance with any of the previous claims **characterised in that** it is comprised in a system comprising a gastric stimulator for stimulation of the gastroenteric system of a subject.

5. Device in accordance with claim 4 **characterised in that** said output signal may be used to control said gastric stimulator in order to initiate, modify and/or stop stimulation of the gastroenteric system of the subj ect.

## Patentansprüche

1. Vorrichtung zum Nachweis der Nahrungsaufnahme in einer Person, **dadurch gekennzeichnet, dass** sie Folgendes umfasst:
- einen Photoplethysmographen (2), der an einem Organ des Verdauungssystems der Person angeordnet sein kann, wobei das Organ der Magen, die Milz, die Bauchspeicheldrüse oder die Leber ist, um die Perfusion des Organs des Verdauungssystems zu messen und ein Ausgangssignal in Abhängigkeit von der Perfusion zu erzeugen, und
- eine Analysevorrichtung, die angeordnet ist, um das Ausgangssignal zu analysieren, um eine Nahrungsaufnahme nachzuweisen.

2. Vorrichtung nach Anspruch 1, **dadurch gekennzeichnet, dass** der Photoplethysmograph am Magen (3) der Person angebracht sein kann.

3. Vorrichtung nach Anspruch 1 oder 2, **dadurch gekennzeichnet, dass** der Photoplethysmograph an einem Band oder Ring, das bzw. der um den Magen der Person herum platziert sein kann, befestigt sein kann, oder der Photoplethysmograph mit Nahtlöchern (25) versehen ist.

4. Vorrichtung nach einem der vorhergehenden Ansprüche, **dadurch gekennzeichnet, dass** sie in einem System enthalten ist, das einen Magenstimulator zur Stimulation des Magen-Darm-Systems einer Person umfasst.

5. Vorrichtung nach Anspruch 4, **dadurch gekennzeichnet, dass** das Ausgangssignal verwendet werden kann, um den Magenstimulator zu steuern, um die Stimulation des Magen-Darm-Systems der Person auszulösen, abzuändern und/oder anzuhalten.

## Revendications

1. Dispositif de détection de la prise de nourriture dans un sujet, **caractérisé en ce qu'**il comprend :
- un photopléthysmographe (2) capable d'être agencé sur un organe du système digestif dudit sujet, ledit organe étant l'estomac, la rate, le pancréas ou le foie, afin de mesurer la perfusion dudit organe du système digestif et de générer un signal de sortie dépendant de ladite perfusion, et
- un dispositif d'analyse agencé pour analyser ledit signal de sortie de façon à détecter la prise de nourriture.

2. Dispositif selon la revendication 1, **caractérisé en ce que** ledit photopléthysmographe peut être raccordé à l'estomac (3) dudit sujet.

3. Dispositif selon la revendication 1 ou 2, **caractérisé en ce que** ledit photopléthysmographe peut être monté sur une bande ou un anneau qui peut être placé(e) autour de l'estomac dudit sujet ou ledit photopléthysmographe est doté de trous de suture (25).

4. Dispositif selon l'une quelconque des revendications précédentes, **caractérisé en ce qu'**il est inclus dans un système comprenant un stimulateur gastrique pour la stimulation du système gastro-entérique d'un sujet.

5. Dispositif selon la revendication 4, **caractérisé en ce que** ledit signal de sortie peut être utilisé pour contrôler ledit stimulateur gastrique afin d'amorcer, de modifier et/ou d'arrêter la stimulation du système gastro-entérique du sujet.
